## Europäisches Patentamt

## European Patent Office

## Office européen des brevets

(11) Veröffentlichungsnummer: **0 328 091**

**A2**

## (12) EUROPÄISCHE PATENTANMELDUNG

(21) Anmeldenummer: **89102210.5**

(22) Anmeldetag: **09.02.89**

(51) Int. Cl.⁴: **A61K 31/275 , A61K 31/195 ,**
**//(A61K31/275,31:19),**
**(A61K31/195,31:19)**

(30) Priorität: **11.02.88 DE 3804141**

(43) Veröffentlichungstag der Anmeldung:
**16.08.89 Patentblatt 89/33**

(84) Benannte Vertragsstaaten:
**ES GR**

(71) Anmelder: **Kaiser, Hans-Gerd**
**Germanenstrasse 44**
**D-5650 Solingen 1(DE)**

(72) Erfinder: **Kaiser, Hans-Gerd**
**Germanenstrasse 44**
**D-5650 Solingen 1(DE)**

(74) Vertreter: **Heiber, Margarita, Dr.**
**Futterstrasse 33**
**D-5600 Wuppertal 2(DE)**

(54) **Wirksubstanz zur Behandlung von Haut- und Schleimhautveränderungen.**

(57) Die Erfindung betrifft eine Wirksubstanz zur topischen Behandlung von Haut- und Schleimhautveränderungen, insbesondere solchen, die durch Stoffwechsel- und Gewebeerkrankungen verursacht sind.
Die Wirksubstanz ist ein Substanzgemisch aus zwei Komponenten a) und b), wobei

a) aus mindestens einer Aminopolycarbonsäure, und/oder ihren Salzen und/oder ihren Derivaten oder aus Gluconsäure, und/oder ihren Salzen und/oder Derivaten und

b) aus mindestens einem Salz und/oder Derivat einer Fruchtsäure besteht.

Die Wirksubstanz führt zum schnellen Abklingen der Symptome unter anderem bei Psoriasis, Akne, Lichen ruber, Neurodermitis, Parodontitis, Gingivitis, Herpes. Sie kann pharmazeutisch und kosmetisch eingesetzt werden.

## Wirksubstanz zur Behandlung von Haut- und Schleimhaut-veränderungen

Die Erfindung geht davon aus, daß bei den meisten Erkrankungen als Folge eine Veränderung des Stoffwechsels eintritt. Bei diesen Stoffwechselveränderungen sind zu einem erheblichen Teil Differenzen im Bereich der Spurenelemente festzustellen. Es ist hinreichend bekannt, daß ein Fehlen der Spurenelemente spezifische Mangelerscheinungen hervorruft. Ein Überangebot löst ebenfalls atypische Erkrankungen aus.

Zur Therapie von Stoffwechselstörungen wie sie etwa bei Schwermetallintoxikationen oder bei Arteriosclerose und verwandten Erkrankungen vorliegen, haben sich Polyaminocarbonsäuren, insbesondere Ethylendiamintetraessigsäure (EDTA) bewährt (B.W. Halstead "The Scientific Basis of EDTA Chelation Therapy", Golden Quill Publ. Inc., Colton 1979, Seiten 21 bis 27).

Die komplexbildende Wirkung der Polyhydroxycarbonsäuren und ihrer Salze, wie etwa die von D-Gluconaten, nutzt man in der Calzium-Therapie und zur Bereitstellung von biologisch verfügbarem Eisen in tonisierenden Mitteln aus (Pharmazeutische Stoff liste, Hrsg. ABDA, Frankfurt/Main "Gluconsäure" S. 84 bis 87).

Fruchtsäuren, insbesonder Citronensäure, fanden bisher Anwendung in Arzneimitteln als Ansäuerungsmittel in Sprudeltabletten, als Geschmackskorrigens, Desinfizienz und zur Magensäuresubstitution (Pharmazeutische Stoffliste, Hrsg.: ABDA, Frankfurt/Main "Citronensäure" S. 86 bis 89).

In der DE-OS 16 17 607 werden Mischungen aus Schwermetallsalzen, einund mehrbasischen Di-Nitrilo-R-carbonsäuren sowie R-Oxy-R-Carbonsäuren angegeben, die bei Rheuma, Virusinfektionen und zur Förderung der Wundheilung physiologische Wirkungen aufweisen.

Zur Behandlung von der Folgen von Stoffwechselstörungen im Bereich der Haut, der Schleimhäute und Hautanhangsgebilde wurden diese Substanzgruppen bislang weder allein, noch in Kombination eingesetzt.

Es wurde nun überraschenderweise gefunden, daß sich, wie in den Ansprüchen beschrieben, mittels einer Kombination von Salzen oder Derivaten von Fruchtsäuren mit Salzen komplexbildender organischer Säuren, wie von Aminopolycarbonsäuren und/oder ihren Salzen und/oder ihren Derivaten oder von einem Salz und/oder Derivat der Gluconsäure die Folgen von Stoffwechsel- und Gewebeerkrankungen an Haut, Schleimhäuten und Hautanhangsgebilden positiv beeinflussen lassen.

So können Überangebote durch komplexbildende Salze eliminiert werden oder aber in eine Form gebracht werden, die eine bessere oder leichtere Verteilung bei einer Unterversorgung ermöglicht.

Die anspruchsgemäßen Formulierungen zeigten ein überraschend weites Wirkungsspektrum. Bei allen nachfolgend beschriebenen Erkrankungen trat in kürzester Zeit Linderung oder Heilung ein. Als sehr wesentlich muß herausgestellt werden, daß die Formulierungen nicht toxisch sind und keinerlei Nebenwirkungen zeigen.

Die erfindungsgemäße Wirksubstanz wurde bei verschiedenen Haut- und Schleimhautveränderungen eingesetzt:

1. Erkrankung durch
   a) Bakterien
z.B. Follikulitis, Furunkel
   b) Viren
z.B. Herpes simplex, Varizellen, Aphthen
   c) Mikroben
z.B. Stomatitis, Paradontitis
   d) Pilze
z.B. Intertrigo

2. Erkrankungen durch physikalische oder chemische Noxen
z.B. Hyperkeratosen, mechanische Hautschäden (u.a. Narben)

3. Erkrankungen durch exogene oder endogene Allergene
z.B. Cheilitis, Kontaktdermatitis durch Pflanzen, Metalle oder Arzneimittel, Psoriasis-Gruppe, Neurodermitis, Lichen ruber planus

4. Erkrankungen durch Stoffwechselstörungen (u.a. toxische)
z.B. Akne vulgaris, Ekzema seborroicum, Hyperhidrosis

5. Erkrankungen durch endokrine Störungen
z.B. Parodontopathien, Fibrosen

Es gibt Anhaltspunkte dafür, daß die erfindungsgemäßen Mittel auch einen zytostatischen Effekt haben können (vgl. 5)

7. Kosmetische Behandlung der Haut.

Anwendungsbeispiele

1. Mit einer Lösung bestehend aus:
20% Ammoniumcitrat
10% Natriumgluconat
5% Natriumlactat
65% $H_2O$
wurden Probanden behandelt. Die Lösung wurde mit Wattestäbchen appliziert.

A) Diagnose: Allergisches Ekzem an beiden Händen
Therapie: a) Cortisonpräparate - keine Besserung
b) beanspruchte Lösung - Hauteffloreszenzen nach 6 Tagen abgeheilt

B) Diagnose: Psoriasis am ganzen Körper (in Hautklinik histologisch nachgewiesen)
Nach 15 Wochen erfindungsgemäßer Behandlung Hautveränderung abgeheilt; Juckreiz nach 3 Tagen beseitigt.

C) Diagnose: Akne vulgaris (seit 7 Jahren)
Durch Behandlung mit erfindungsgemäßer Lösung nach 2 Wochen aknefrei

2. Mit einer Lösung bestehend aus :
20% Ammoniumcitrat
10% (Ethylendinitrilotetraessigasäure-Dinatriumsalz) Dihydrat
5% Natriumlactat
65% H$_2$O
wurden Probanden in der gleichen Weise behandelt. Der Heilerfolg war deckungsgleich mit dem unter Beispiel 1. In einigen Fällen wurde das Substanzgemisch auch als Gel appliziert, um eine bessere Haftung an den zu behandelnden Stellen zu erzielen. Hierzu wurde die Lösung mit hochdisperser Kieselsäure in Salbenkonsistenz gebracht.
Beobachtet wurde bei den behandelten Probanden eine starke Straffung der oberen Hautpartien, so daß davon auszugehen ist, daß dieses Präparat auch als Kosmetikum einzusetzen ist.

3. Lösung bestehend aus
10 Gew% Citronensäure
5 Gew% Ethylendinitrilotetraessigsäure
85 Gew% H$_2$O
pH-Wert auf 9 mit KOH eingestellt.
Mit dieser Lösung wurden 6 Probanden behandelt.
Diagnose: seborrhoisches Ekzem
Therapie: Die befallenen Stellen wurden 2 - 3 mal tägl. leicht eingerieben.
Hauteffloreszenzen nach 8 Tagen abgeheilt.

4. Lösung bestehend aus:
0,1 Gew% Citronensäure
0,5 Gew% (Ethylendinitrilotetraessigsäure-Dinatriumsalz) Dihydrat
99,4 Gew% H$_2$O
pH-Wert auf 5,2 mit NaOH eingestellt.
Mit dieser Lösung wurden 3 Probanden (Kleinkinder) behandelt.
Diagnose: Neurodermitis
Therapie: Die befallenen Stellen wurden mit der Lösung 3 mal täglich leicht eingerieben.
Linderung des typischen Juckreizes bereits nach 8 Stunden. Abheilung nach ca. 12 Tagen.

5. Lösung bestehend aus:
10 Gew% Äpfelsäure
5 Gew% (Ethylendinitrilotetraessigsäure-Dinatriumsalz) Dihydrat
85 Gew% H$_2$O
pH-Wert mit Ammoniak auf 5,2 eingestellt.
Mit dieser Lösung wurden 10 Probanden behandelt.
Diagnose: Gingivitis simplex
Therapie: Einpinseln der befallenen Stellen. Bereits nach der ersten Behandlung wurde ein Abklingen der entzündlichen Erscheinungen beobachtet. Abheilung bei 2 mal tägl. Anwendung nach ca. 7 Tagen.

6. Eine Lösung bestehend aus:
10 Gew% Kaliumcitrat
3 Gew% Cyclohexylen-(1,2)-dinitrilotetraessigsäure (Monohydrat)
87 Gew% H$_2$O
wurde mittels hoch disperser Kieselsäure in Salbenkonsistenz gebracht.
Mit dieser Salbe wurden 4 Probanden behandelt.
Diagnose: Stark nässende und juckende Ekzeme
Therapie: 2 mal tägl. Auftragen der Salbe. Hautveränderungen je nach Schweregrad nach 4 - 9 Wochen abgeheilt.

7. Eine Lösung bestehend aus:
20 Gew% Kaliumcitrat
5 Gew% Diethylentriaminpentaessigsäure, 3-Aza-3-(Carboxymethyl) pentamethylendinitrilotetraessigsäure
75 Gew% H$_2$O
wurde mit hoch disperser Kieselsäure in Salbenkonsistenz gebracht.
Mit dieser Salbe wurde ein Proband behandelt.
Diagnose: Wespenstich mit lokaler Infektion, allergischer Hautreaktion und Schwellung des linken Unterschenkels lateral.
Therapie: 2 mal tägl. Auftragen der Salbe. Juckreiz nach 10 Min. abgeklungen. Nach 3 Tagen war die gesamte allergische Hautreaktion abgeklungen und der lokale Infektionsherd an der Einstichstelle verheilt. Nach Absetzen der Salbe kein Rezidiv.

8. Eine Lösung bestehend aus:
5 Gew% Kaliumcitrat
0,1 Gew% Bis-(aminoethyl)-glycolether-N,N,N´,N´-tetraessigsäure (3,6 Dioxaoctamethylen-dinitrilo-tetraessigsäure)
94,9 Gew% H$_2$O
wurde mit Talkum auf Salbenkonsistenz gebracht.
Mit dieser Salbe wurden 4 Patienten behandelt.
Diagnose: Starke Kontaktallergie
Therapie: 1 - 2 mal tägl. Auftragen der Salbe an den befallenen Stellen. Hauteffloreszenzen nach 4 - 6 Tagen abgeheilt.

9. Mit einer Lösung bestehend aus:
10 Gew% Ammoniumcitrat
5 Gew% Natriumgluconat
85 Gew% H$_2$O
wurden 9 Probanden behandelt:
Diagnose: Tonsillitis

Therapie: Die Ausgangslösung wurde von den Probanden selbst mit Leitungswasser auf 1 : 1 - 1 : 4 verdunnt und 3 - 4 mal tägl. gegurgelt. Nach 2 - 3 Tagen waren die Probanden beschwerdefrei.

10. Mit einer Lösung aus:
20 Gew% Ammoniumcitrat
10 Gew% Ethylendinitrilotetraessigsäure-Dinatriumsalz -Dihydrat
70 Gew% W/O Emulsion
wurden 3 Probanden behandelt.
Diagnose: Rosacea
Therapie: 2 mal tägliches Auftragen der Emulsion. Bereits nach 4 Tagen zeigt sich ein deutlicher Rückgang der Effloreszenzen. Nach Behandlung über 4 Wochen und Absetzen der Salbe kein Rezidiv.

11. Mit einer Lösung bestehend aus:
20 Gew% Ammoniumcitrat
10 Gew% (Ethylendinitrilotetraessigsäure-Dinatriumsalz) -Dihydrat
70 Gew% H₂O
wurden 32 Probanden behandelt.
Diagnose: Starke Parodontitis
Therapie: Appliziert wurde durch 2 - 3 mal tägl. Auftragen mit Wattestäbchen. Abheilung nach 10 -14 Tagen je nach Schwere der Erscheinung.

12. Lösung bestehend aus:
20 Gew% Ammoniumcitrat
10 Gew% (Ethylendinitrilotetraessigsäure-Dinatriumsalz) -Dihydrat
70 Gew% H₂O
Diese Lösung wurde mittels hoch disperser Kieselsäure auf Salbenkonsistenz eingestellt.
Behandelt wurden hiermit 8 Probanden.
Diagnose: Lichen ruber planus
Therapie: Die zu behandelnden Partien wurden 1 - 2 mal tägl. mit der Salbe eingestrichen. Deutliche Besserung bereits nach der 3. Behandlung. Abheilung ohne Rezidiv nach ca. 4 Monaten.

13. Lösung bestehend aus:
3 Gew% Ammoniumtartrat
3 Gew% (Ethylendinitrilotetraessigsäure-Dinatriumsalz) Dihydrat
94 Gew% H₂O
Mit dieser Lösung wurde 1 Proband behandelt.
Diagnose: Herpes simplex
Therapie: Appliziert wurde 2 - 3 mal tägl. mit Wattestäbchen. In allen Fällen Abheilung nach 3 -4 Tagen.

14. Lösung bestehend aus:
0,1 Gew% Nitrilotriessigsäure
0,2 Gew% Fumarsäure-Dinatriumsalz
50,0 Gew% Ethylalkohol
49,7 Gew% H₂O
Diese Lösung wurde mittels hochdisperser Kieselsäure auf Salbenkonsistenz eingestellt.
Behandelt wurden 11 Probanden.
Diagnose: Kontaktdermatitis

Therapie: Durch Einreiben der befallenen Stellen Rückgang der Effloreszenzen nach ca. 8 - 12 Std.; Abheilung nach 3 - 4 Tagen.

**Ansprüche**

1. Substanzgemisch aus
a) mindestens einer Aminopolycarbonsäure mit N-Carboxymethyl-Gruppen und/oder ihren Salzen und/oder ihren Derivaten
b) mindestens einem Salz und/oder Derivat einer Fruchtsäure
als Wirksubstanz zur
Behandlung von Haut- und Schleimhautveränderungen.

2. Substanzgemisch nach Anspruch 1, **dadurch gekennzeichnet, daß** das Gewichtsverhältnis von a) : b) in dem Gemisch in dem Bereich von 1 : 4 bis 4 : 1 liegt, gegebenenfalls zur Konfektionierung übliche physiologisch unbedenkliche Hilfsmittel zugesetzt sind und der pH-Wert auf einen Wert im Bereich von 3,5 bis 11 eingestellt ist.

3. Substanzgemisch nach Ansprüchen 1 oder 2, **dadurch gekennzeichnet, daß**
a) aus Ethylendiamintetraessigsäure, Nitrilotriessigsäure, Cyclohexylen-(1,2)-dinitrilotetraessigsäure, Diethylentriaminpentaessigsäure (3-Aza-3-(Carboxymethyl)-pentamethylendinitrilotetraessigsäure), Bis-(aminoethyl)-glycolether-N,N,N′,N′-tetraessigsäure, (3,6 Dioxamethylendinitrilotetraessigsäure) und/oder ihren Salzen und/oder deren Derivaten besteht.

4. Substanzgemisch nach Ansprüchen 1 bis 3, **dadurch gekennzeichnet, daß**
b) aus einem Salz oder Derivat der Citronensäure, vorzugsweise ihrem Ammoniumsalz besteht.

5. Substanzgemisch nach Ansprüchen 1 bis 4, **dadurch gekennzeichnet, daß** das Gemisch von a) und b) in einer Konfektionierung eingesetzt wird, in der a) und b) in einer Konzentration von 0,1 bis 50 Gew% bezogen auf die Gesamtmasse des jeweiligen Präparats vorliegen.

6. Subsstanzgemisch aus
a) mindestens einem Salz und/oder Derivat der Gluconsäure und
b) mindestens einem Salz und/oder Derivat einer Fruchtsäure
als Wirksubstanz in der Herstellung von topischenn Präparaten zur Behandlung von Haut- und Schleimhautveränderungen.

7. Substanzgemisch nach Anspruch 6, **dadurch gekennzeichnet, daß** das Gewichtsverhältnis von a) : b) im Bereich von 1 : 4 bis 4 : 1 liegt, gegebenenfalls zur Konfektio nierung übliche pharmazeutische Hilfsstoffe zugesetzt sind und der pH-Wert auf einen Wert im Bereich von 3,5 bis 11 eingestellt ist.

8. Substanzgemisch nach Ansprüchen 6 oder 7,

**dadurch gekennzeichnet, daß** b) aus einem Salz oder Derivat der Citronensäure, vorzugsweise ihrem Ammoniumsalz, besteht.

9. Substanzgemisch nach Ansprüchen 6 bis 8,

**dadurch gekennzeichnet, daß** das Gemisch aus a) und b) in einer Konfektionierung eingesetzt wird, in der a) und b) in einer Konzentration von 0,1 bis 50 Gew% bezogen auf die Gesamtmasse des jeweiligen Präparates vorliegen.

10. Kosmetische Zubereitung,

**dadurch gekennzeichnet, daß** sie neben zur Konfektionierung üblichen Zusatzstoffen und gegebenenfalls weiteren Wirkstoffen das Substanzgemisch gemäß Ansprüchen 1 bis 5 und/oder 6 bis 10 enthält.

11. Anwendung von Substanzgemisch nach Ansprüchen 1 bis 5 und/oder 6 bis 10 in der kosmetischen Behandlung.

12. Verwendung des Substanzgemischs nach Ansprüchen 1 bis 5 und/oder 6 bis 10 zur Herstellung pharmazeutischer Präparate zur Behandlung von Haut- und Schleimhautveränderungen.